# EUROPEAN PATENT APPLICATION

(11) **EP 4 542 714 A1**
(43) Date of publication of application: **23.04.2025**
(21) Application number: 23823543.6
(22) Date of filing: 27.04.2023
(51) Int. Cl.: H01M 10/0568, H01B 1/06, H01M 4/62, H01M 10/052, H01M 10/056

(54) **ALKALI METAL ION CONDUCTOR, ALKALI METAL ION BATTERY, AND ALKALI METAL ION CONDUCTOR PRODUCTION METHOD**

(30) Priority: 17.06.2022 JP 2022098343
(71) Applicant: TOYOTA JIDOSHA KABUSHIKI KAISHA, Toyota-shi Aichi 471-8571 (JP)
(72) Inventor: MINAMI, Keiichi, Toyota-shi, Aichi 471-8571 (JP)
(74) Representative: D Young & Co LLP
(86) International application number: PCT/JP2023/016729
(87) International publication number: WO 2023/243240

(57) **Abstract**

Disclosed is an alkali metal ion conductor which behaves as a liquid at a low temperature. The alkali metal ion conductor of the present disclosure is an alkali metal ion conductor comprising a salt, wherein the salt comprises a first cation, a second cation, and a first anion, the first cation is a tetraalkylammonium ion having an alkyl chain length of 5 or more, the second cation is an alkali metal ion, and the first anion is at least one anion selected from the group consisting of a bromine ion, a chlorine ion, and a hydrogen sulfate ion.

## Description

### FIELD

The present application discloses an alkali metal ion conductor, an alkali metal ion battery, and a method for manufacturing an alkali metal ion conductor.

### BACKGROUND

Patent Document 1 discloses, as a solid electrolyte constituting a solid electrolyte layer of a secondary battery, those comprising a lithium salt, a sulfide solid electrolyte, and an organic electrolyte having a predetermined elastic modulus ratio. The organic electrolyte disclosed in Patent Document 1 has the property that the ratio of the storage elastic modulus to the loss elastic modulus decreases to less than 1 when heated. In Patent Document 1, by heating the solid electrolyte layer comprising a lithium salt, a sulfide solid electrolyte, and an organic electrolyte, the organic electrolyte changes from behaving as a solid to behaving as a liquid with the rise of the temperature, and the organic electrolyte becomes compatible with the sulfide solid electrolyte, leading to an improvement in ionic conductivity. Meanwhile, Patent Documents 2 to 4 disclose various salts as additives to an electrolytic solution.

### [CITATION LIST]

### [PATENT LITERATURE]

[PTL 1] JP 2020-198270 A
[PTL 2] JP 2018-133285 A
[PTL 3] JP 2004-103558 A
[PTL 4] JP H2-114464 A

### SUMMARY

### [TECHNICAL PROBLEM]

The organic electrolyte disclosed in Patent Document 1 behaves as a liquid at a high temperature. When such organic electrolyte is used in various electrochemical devices, heating of the devices may cause an increase in manufacturing cost, and heating of the devices may cause deterioration of the performance of the devices. In view of these problems, the present application discloses an alkali metal ion conductor that behaves as a liquid at a low temperature.

### [SOLUTION TO PROBLEM]

The present application discloses the following plurality of aspects as means for solving the above problems.

### <Aspect 1>

An alkali metal ion conductor comprising a salt, wherein
the salt comprises a first cation, a second cation, and a first anion,
the first cation is a tetraalkylammonium ion having an alkyl chain length of 5 or more,
the second cation is an alkali metal ion, and
the first anion is at least one anion selected from the group consisting of a bromine ion, a chlorine ion, and a hydrogen sulfate ion.

### <Aspect 2>

The alkali metal ion conductor according to Aspect 1, wherein
the alkali metal ion is a lithium ion.

### <Aspect 3>

The alkali metal ion conductor according to Aspect 1 or 2, wherein
the salt comprises a second anion, and
the second anion is at least one anion selected from the group consisting of a sulfonylamide anion and a complex ion containing H.

### <Aspect 4>

An alkali metal ion battery comprising a positive electrode, an electrolyte layer, and a negative electrode, wherein
at least one of the positive electrode, the electrolyte layer and the negative electrode comprises the alkali metal ion conductor according to any one of Aspects 1 to 3.

### <Aspect 5>

A method for manufacturing an alkali metal ion conductor, which comprises:
mixing at least one tetraalkylammonium salt with at least one alkali metal salt, wherein
the tetraalkylammonium salt has an alkyl chain length of 5 or more, and comprises at least one first anion selected from the group consisting of a bromine ion, a chlorine ion, and a hydrogen sulfate ion.

### <Aspect 6>

The manufacturing method according to Aspect 5, wherein
the alkali metal salt is a lithium salt.

### <Aspect 7>

The manufacturing method according to Aspect 5 or 6, wherein the alkali metal salt comprises at least one second anion selected from the group consisting of a sulfonylamide anion and a complex ion containing H.

### [EFFECTS OF INVENTION]

The alkali metal ion conductor of the present disclosure has alkali metal ionic conductivity and behaves as a liquid at a low temperature.

### BRIEF DESCRIPTION OF DRAWINGS

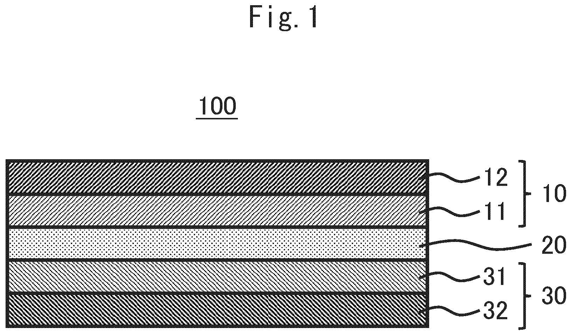

FIG. 1 schematically shows the configuration of an alkali metal ion battery.

### DESCRIPTION OF EMBODIMENTS

### 1. Alkali Metal Ion Conductor

The alkali metal ion conductor of the present disclosure includes a salt. The salt includes a first cation, a second cation, and a first anion. The first cation is a tetraalkylammonium ion having an alkyl chain length of 5 or more. The second cation is an alkali metal ion. The first anion is at least one anion selected from the group consisting of a bromine ion, a chlorine ion, and a hydrogen sulfate ion.

### 1.1 Cation and Anion

The salt may be any one containing the above cations and anions. The salt may be a mixed salt containing the above cations and anions. The mixed salt refers to those obtained by mixing a plurality of types of salts, and refers to a salt obtained by combining a plurality of types of cation and/or a plurality of types of anions.

### 1.1.1 First Cation

The salt constituting the alkali metal ion conductor of the present disclosure contains, as a first cation, a tetraalkylammonium ion having an alkyl chain length of 5 or more. The first cation has a structure represented by the following chemical formula (1). According to new findings of the present inventors, when the first cation has an alkyl chain length of 5 or more and also constitutes the salt together with the below-mentioned second cation and first anion, the melting temperature of the salt is significantly lowered: 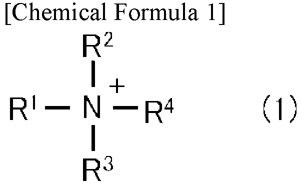 wherein R¹ , R² , R³ , and R⁴ are each independently an alkyl group having an alkyl chain length of 5 or more. R¹ , R² , R ³ , and R⁴ may be alkyl groups identical to each other.

"Alkyl chain length" refers to the maximum length of the carbon chain of the alkyl group. In other words, the alkyl group constituting the first cation may have a longest chain (long chain) having 5 or more carbon atoms. The alkyl group constituting the first cation may be a linear alkyl group having an alkyl chain length of 5 or more, or may be an alkyl group that has a long chain having an alkyl chain length of 5 or more and a branched chain connected to the long chain.

The alkyl chain length of the first cation is 5 or more, or may be 6 or more, 7 or more, or 8 or more. The upper limit of the alkyl chain length is not particularly limited, as long as the alkyl chain length can exist as a cation. The alkyl chain length may be, for example, 20 or less, 15 or less, or 10 or less. Specific examples of the first cation having a linear alkyl group include a tetrapentylammonium ion (tetraamylammonium ion), a tetrahexylammonium ion, a tetraheptylammonium ion, a tetraoctylammonium ion, a tetranonylammonium ion, and a tetradecylammonium ion. The first cation may be of only one type, or of a combination of two or more types thereof.

### 1.1.2 Second Cation

The salt constituting the alkali metal ion conductor of the present disclosure contains, as a second cation, an alkali metal ion. The type of the alkali metal ion may be determined depending on the type of a carrier ion to be conducted. For example, when the alkali metal ion conductor of the present disclosure is one used in a lithium ion battery, the alkali metal ion as the second cation may be a lithium ion. In other words, since the alkali metal salt contains the same type of cation as the carrier ion in an alkali metal ion battery, the performance of the alkali metal ion battery is more easily enhanced. The second cation may be of only one type, or of a combination of two or more types thereof.

### 1.1.3 Other Cations

The cation constituting the salt may be composed only of the above first cation and second cation, or may contain other cations within a range in which a desired effect is exhibited. Examples of other cations include ions containing poor metallic element. Examples of poor metal include Al, Ga and the like. From the viewpoint that the effect by the alkali metal ion conductor of the present disclosure is further enhanced, the total ratio of the above first cation and second cation to the entire cation constituting the salt may be 50 mol% or more, 60 mol% or more, 70 mol% or more, 80 mol% or more, 90 mol% or more, 95 mol% or more, 99 mol% or more, or 100 mol%.

### 1.1.4 Molar Ratio for Cations

The molar ratio of the first cation to the second cation constituting the salt is not particularly limited. When the salt contains the first cation and the second cation, the melting point of the salt is lowered as compared with a case where each of them is contained alone. From the viewpoint of significantly lowering the melting point of the salt and further enhancing the alkali metal ionic conductivity, the molar ratio of the second cation to the first cation (second cation/first cation) may be 0.05 or more and 19.0 or less. The molar ratio may be 0.1 or more, 0.2 or more, 0.3 or more, 0.4 or more, 0.5 or more, 0.6 or more, 0.7 or more, 0.8 or more, 0.9 or more, or 1.0 or more, and may be 10.0 or less, 9.5 or less, 9.0 or less, 8.5 or less, 8.0 or less, 7.5 or less, 7.0 or less, 6.5 or less, 6.0 or less, 5.5 or less, or 5.0 or less. In the alkali metal ion conductor of the present disclosure, even if the molar ratio of the second cation to the first cation constituting the salt is as high as 1.0 or more (for example, the concentration of alkali metal ions accounts for 50 mol% or more of all cations), the melting temperature of the salt can be near room temperature.

### 1.1.5 First Anion

The salt constituting the alkali metal ion conductor of the present disclosure contains, as the first anion, at least one anion selected from the group consisting of a bromine ion, a chlorine ion, and a hydrogen sulfate ion. According to new findings of the present inventors, when the salt constituting the alkali metal ion conductor contains the above first anion, the melting temperature of the salt is specifically lowered. In other words, the salt behaves as a liquid at a low temperature.

### 1.1.6 Second Anion

The salt constituting the alkali metal ion conductor of the present disclosure may contain a second anion. The second anion is an anion that is different from the first anion. The second anion may be, for example, at least one of an anion selected from the group consisting of a sulfonylamide anion and a complex ion containing H.

Examples of the sulfonylamide anion include symmetrical anions such as a bistrifluoromethanesulfonylamide anion (TFSA anion, (CF₃ SO₂)₂N⁻) and a fluorosulfonylamide anion (FSA anion, (FSO₂)₂N⁻), and asymmetrical anions such as a fluorosulfonyl(trifluoromethanesulfonyl)amide anion (FTA anion, FSO₂ (CF₃ SO₂)N⁻). The sulfonylamide anion may be of only one type, or of a combination of two or more types thereof. Of the above sulfonylamide anions, a TFSA anion has low polarity and has particularly low reactivity with other materials. For example, when the salt constituting the alkali metal ion conductor of the present disclosure contains the sulfonylamide anion, if the sulfonylamide anion is the TFSA anion, the reaction between the salt and the below-mentioned sulfide solid electrolyte is more easily suppressed.

The complex ion containing H may contain, for example, an element M containing at least one of a non-metallic element and a metallic element, and H bonded to the element M. In the complex ion containing H, an element M as a central element and H surrounding the element M may also be bonded to each other via a covalent bond. The complex ion containing H may also be represented by (Mₘ Hₙ )^{α -}. In this case, m is any positive number, and n and α can take any positive number according to m, the valence of the element M and the like. The element M may be any non-metallic or metallic element capable of forming a complex ion. For example, the element M may include at least one of B, C, and N as the non-metallic element, and may also include B. For example, the element M may include at least one of Al, Ni, and Fe as the metallic element. In particular, when the complex ion contains B or contains C and B, higher ionic conductivity is easily ensured. Specific examples of the complex ion containing H include (CB₉H₁₀)⁻, (CB₁₁H₁₂)⁻, (B₁₀H₁₀)²⁻ , (B₁₂H₁₂)²⁻, (BH₄)⁻, (NH₂)⁻, (AlH₄)⁻, and combinations thereof. In particular, when using (CB₉H₁₀)⁻, (CB₁₁H₁₂)⁻, or a combination thereof, higher ionic conductivity is easily ensured.

### 1.1.7 Other Anions

The anion constituting the salt may be of only of the above first anion, or may be of only of the above first anion and second anion, or may contain other anions within a range in which a desired effect is exhibited. From the viewpoint of further enhancing the effect by the alkali metal ion conductor of the present disclosure, the total ratio of the first anion and the second anion in the entire anion constituting the salt may be 50 mol% or more, 60 mol% or more, 70 mol% or more, 80 mol% or more, 90 mol% or more, 95 mol% or more, 99 mol% or more, or 100 mol%.

### 1.1.8 Molar Ratio for Anions

The molar ratio of the first anion to the second anion is not particularly limited. For example, the molar ratio of the second anion to the first anion (second anion/first anion) may be 0.05 or more and 19.0 or less. The molar ratio may be 0.1 or more, 0.2 or more, 0.3 or more, 0.4 or more, or 0.5 or more, and may be 10.0 or less, 9.5 or less, 9.0 or less, 8.5 or less, 8.0 or less, 7.5 or less, 7.0 or less, 6.5 or less, 6.0 or less, 5.5 or less, or 5.0 or less.

### 1.2 Melting Temperature of Salt

The salt containing the above first and second cations and first anion behaves as a liquid at a low temperatures, i.e., melts at a low temperature. In other words, the salt behaves like an ionic liquid. The melting temperature of the salt constituting the alkali metal ion conductor of the present disclosure may be, for example, 40°C or lower, 30°C or lower, or 20°C or lower.

### 1.3 Viscosity of Salt

As mentioned above, the salt constituting the alkali metal ion conductor of the present disclosure melts at a low temperature. The molten salt preferably has a high viscosity. For example, the viscosity of the salt at 25°C may be 10,000 mPa·s or more. If the salt has such a viscosity, when the salt is applied to a battery, the salt easily remain inside electrodes and an electrolyte layer.

### 1.4 Components other than Salt

The alkali metal ion conductor of the present disclosure may be composed only of the above salt, or may be those obtained by combining the above salt with other electrolytes or the like. For example, the alkali metal ion conductor of the present disclosure may be a combination of the above salt and a solid electrolyte. For example, excellent ionic conductivity can be ensured by filling gaps and cracks in the solid electrolyte with the salt. It is possible to employ, as the solid electrolyte, for example, any one used as a solid electrolyte of a battery. The solid electrolyte may be a sulfide solid electrolyte containing at least an alkali metal and S as constituent elements. In this case, the alkali metal constituting the solid electrolyte may be determined depending on the type of carrier ion to be conducted. When the alkali metal ion conductor of the present disclosure is one applied to a lithium ion battery, the sulfide solid electrolyte may contain lithium as an alkali metal. In particular, the sulfide solid electrolyte containing at least Li, S and P as a constituent element has high performance, and the sulfide solid electrolyte based on Li₃PS₄ skeleton and containing at least one or more types of halogens also has high performance. An exemplary solid-state electrolyte may include Li2S-P2S5, Li2S-SiS2, LiI-Li2S-SiS₂, LiI-Si₂S-P₂S₅, Li₂S-P₂S₅-LiI-LiBr, LiI-Li2S-P2S5, LiI-Li₂S-P₂O₅, LiI-Li₃PO₄-P₂S₅, Li₂S-P₂S₅-GeS₂ or the like. The sulfide solid electrolyte may be an amorphous or a crystal. The sulfide solid electrolyte may be, for example, particulate. Only one type of sulfide solid electrolyte may be used alone, or two or more types thereof may be used in combination.

When the alkali metal ion conductor of the present disclosure includes a sulfide solid electrolyte together with the above salt, the mass ratio of the sulfide solid electrolyte to the salt is not particularly limited. For example, the sum of the sulfide solid electrolyte and the salt as 100% by mass, sulfide solid electrolyte ratio may be 40% by mass or more and less than 100% by mass, 50% by mass or more and less than 100% by mass, 60% by mass or more and less than 100% by mass, 70% by mass or more and less than 100% by mass, or 80% by mass or more and less than 100% by mass, and the ratio of the salt may be more than 0% by mass and 60% by mass or less, more than 0% by mass and 50% by mass or less, more than 0% by mass and 40% by mass or less, more than 0 and 30% by mass or less, or more than 0% by mass and 20% by mass or less.

The alkali metal ion conductor of the present disclosure may be those in which the above alkali metal salt, an optional sulfide solid electrolyte, and other components are combined. Other components may be appropriately determined depending on the specific application of the alkali metal ion conductor. For example, when an alkali metal ion conductor is used as an electrode material of an alkali metal ion battery, an active material, a conductive aid, a binder, and the like may be combined together with an alkali metal ion conductor. When an alkali metal ion conductor is used as a material constituting an electrolyte layer of a secondary battery, a binder or the like may be combined together with an alkali metal ion conductor. Note that the alkali metal ion conductor may include the above salt, an optional sulfide solid electrolyte, and other electrolytes (a solid electrolyte other than the sulfide solid electrolyte or a liquid electrolyte). Further, the alkali metal ion conductor of the present disclosure may contain various additives.

### 1.5 Applications

The alkali metal ion conductor of the present disclosure may be employed as alkali metal ion conducting materials in various electrochemical devices. According to the alkali metal ion conductor of the present disclosure, for example, when the above salt is added as an additive to a component composition that could not previously be an ionic liquid, there is a possibility that the liquid state is maintained at room temperature by lowering the melting point. It is also possible to increase the concentration of the alkali metal salt in an electrolytic solution by adding the above salt to the electrolytic solution or the like, which may improve the electrochemical stability and alkali ion transport properties of the electrolyte. An alkali metal ion battery will be described in detail below as an example of an electrochemical device to which the alkali metal ion conductor of the present disclosure is applied.

### 2. Alkali Metal Ion Battery

As shown in FIG. 1, an alkali metal ion battery 100 according to an embodiment comprises a positive electrode 10, an electrolyte layer 20 and a negative electrode 30. Here, at least one of the positive electrode 10, the electrolyte layer 20 and the negative electrode 30 includes the above alkali metal ion conductor of the present disclosure. As mentioned above, the salt constituting the alkali metal ion conductor of the present disclosure has a low temperature (melting temperature) at which it behaves as a liquid. Therefore, for example, during the manufacture of a battery, since the salt fills gaps in electrodes and an electrolyte layer, the filling ratio in the electrodes and electrolyte layer easily increases. Even if cracks or peeling occur in the electrodes or electrolyte layer during use of the battery, the cracked or peeled areas can be filled with the salt to eliminate gaps caused by the cracks or peeling, thus making it difficult to cause interruption of the ion conduction path. In this regard, the alkali metal ion conductor of the present disclosure is included in at least one of the positive electrode 10, the electrolyte layer 20 and the negative electrode 30 of the alkali metal ion battery 100, so that the performance of the alkali metal ion battery 100 is easily enhanced. Note that, although the above alkali metal ion conductor of the present disclosure may be included as a liquid in the alkali metal ion battery 100, a solid electrolyte, other liquid electrolytes or a liquid additive may be used together therewith. The alkali metal ion battery 100 may be, for example, those including a solid electrolyte and a liquid, and those including a solid electrolyte, and the alkali metal ion conductor of the present disclosure that exist around the solid electrolyte.

### 2.1 Positive Electrode

As shown in FIG. 1, the positive electrode 10 according to an embodiment may include a positive electrode active material layer 11 and a positive electrode current collector 12, and in this case, the positive electrode active material layer 11 may include the above alkali metal ion conductor.

### 2.1.1 Positive Electrode Active Material Layer

The positive electrode active material layer 11 includes a positive electrode active material, and may further optionally include an electrolyte, a conductive aid, a binder, and the like. Further, the positive electrode active material layer 11 may contain various additives. When the positive electrode active material layer 11 includes the alkali metal ion conductor of the present disclosure as an electrolyte, the positive electrode active material layer 11 includes a positive electrode active material in addition to the alkali metal ion conductor, and may further optionally include other electrolytes, conductive aids, binders, and various additives. The content of each of the positive electrode active material, the electrolyte, the conductive aid, the binder, and the like in the positive electrode active material layer 11 may be appropriately determined according to the battery performance. For example, taking the entire positive electrode active material layer 11 (entire solid content) as 100% by mass, the content of the positive electrode active material may be 40% by mass or more, 50% by mass or more, or 60% by mass or more, and may be less than 100% by mass or 90% by mass or less. The shape of the positive electrode active material layer 11 is not particularly limited, and may be, for example, a sheet shape having a substantially planar surface. Thickness of the positive active material layer 11 is not particularly limited, and may be for example, 0.1 µm or more, 1 µm or more, 10 µm or more or 30 µm or more, and may be 2 mm or less, 1 mm or less, 500 µm or less or 100 µm or less.

Those known as the positive electrode active material of the alkali metal ion battery may be used as the positive electrode active material. Of known active materials, a material having a potential (charge/discharge potential) for occluding and releasing a predetermined ion and exhibiting a more noble potential than that of a negative electrode active material described later can be used as a positive electrode active material. For example, when constituting a lithium ion battery, various lithium-containing composite oxides such as lithium cobaltate, lithium nickelate, lithium manganate, lithium manganese nickel cobaltate, a spinel-based lithium compound, and oxide-based active materials other than the lithium-containing composite oxides may be used as the positive electrode active material, or a sulfur-based active material such as a single substance sulfur or a sulfur compound may be used. Only one type of positive electrode active material may be used alone, or two or more types thereof may be used in combination. The positive electrode active material may be, for example, particulate, and the size thereof is not particularly limited. The particles of the positive electrode active material may be solid particles, and may be hollow particles, and may be particles having voids. The particles of the positive electrode active material may be primary particles or secondary particles in which a plurality of primary particles are aggregated. The mean particle diameter of the particles of the positive active material may be 1 nm or more, 5 nm or more, or 10 nm or more, and may be 500 µm or less, 100 µm or less, 50 µm or less, or 30 µm or less.

The surface of the positive electrode active material may be coated with a protective layer containing an ion conductive oxide. In other words, the positive electrode active material layer 11 may include a composite comprising the above positive electrode active material and a protective layer provided on the surface thereof. Thus, a reaction between the positive electrode active material and sulfide (e.g., the above sulfide solid electrolyte or the like) is easily suppressed. When the battery is a lithium-ion battery, an ion conductive oxide that covers and protects the surface of the positive active material includes, for example, Li3BO3, LiBO2, Li2CO3, LiAlO2, Li4SiO4, Li2SiO3, Li3PO4, Li2SO4, Li2TiO3, Li₄Ti₅O₁₂, Li₂Ti₂O₅, Li2ZrO3, LiNbO₃, Li₂MoO₄, Li2WO4. The coverage ratio (area ratio) of the protective layer may be, for example, 70% or more, 80% or more, or 90% or more. The thickness of the protective layer may be, for example, 0.1 nm or more or 1 nm or more, and may be 100 nm or less or 20 nm or less.

The electrolyte that may be contained in the positive electrode active material layer 11 may be a solid electrolyte, a liquid electrolyte (electrolytic solution), or a combination thereof.

The solid electrolyte may be any known solid electrolyte for an alkali metal ion battery. As for the solid electrolyte other than the above alkali metal ion conductor of the present disclosure, the one well known as a solid electrolyte of a secondary battery may be used. The solid electrolyte may be an inorganic solid electrolyte or an organic polymer electrolyte. In particular, the inorganic solid electrolyte is excellent in ionic conductivity and heat resistance. As the inorganic solid electrolyte, for example, an oxide solid electrolyte such as lithium lanthanum zirconate, LiPON, Li_{1+X}Al_{X}Ge_{2-X}(PO₄)₃, Li-SiO based glass, or Li-Al-S-O based glass can be exemplified in addition to the above sulfide solid electrolyte. In particular among them, a sulfide solid electrolyte, more particular, a sulfide solid electrolyte containing at least Li, S and P as constituent elements has high performance. The solid electrolyte may be amorphous or crystalline. The solid electrolyte may be, for example, particulate. Only one type of solid electrolyte may be used alone, or two or more types thereof may be used in combination.

The electrolytic solution may contain, for example, an alkali metal ion as a carrier ion. The electrolytic solution may be, for example, a non-aqueous electrolytic solution. For example, as an electrolytic solution, a solution obtained by dissolving an alkali metal salt in a carbonate-based solvent at a predetermined concentration can be used. Examples of the carbonate-based solvents include fluoroethylene carbonate (FEC), ethylene carbonate (EC) and dimethyl carbonate (DMC). Examples of the alkali metal salt include hexafluorophosphate and the like.

Examples of the conductive aid which may be contained in the positive electrode active material layer 11 include carbon materials such as vapor-grown carbon fibers (VGCF), acetylene black (AB), Ketjen black (KB), carbon nanotubes (CNT), and carbon nanofibers (CNF); and metallic materials such as nickel, aluminum, and stainless steel. The conductive aid may be, for example, particulate or fibrous, and the size thereof is not particularly limited. Only one type of the conductive aid may be used alone, or two or more types thereof may be used in combination.

Examples of the binder which may be contained in the positive electrode active material layer 11 include a butadiene rubber (BR) based binder, a butylene rubber (IIR) based binder, an acrylate butadiene rubber (ABR) based binder, a styrene butadiene rubber (SBR) based binder, a polyvinylidene fluoride (PVdF) based binder, a polytetrafluoroethylene (PTFE) based binder, a polyimide (PI) based binder, a polyacrylic acid-based binder, and the like. Only one type of binder may be used alone, or two or more types thereof may be used in combination.

### 2.1.2 Positive electrode Current Collector

As shown in FIG. 1, the positive electrode 10 may include a positive electrode current collector 12 in contact with the above positive electrode active material layer 11. The positive electrode current collector 12 can employ any of common ones as a positive electrode current collector of a battery. Further, the positive electrode current collector 12 may be a foil, a plate, a mesh, a punching metal, a foam, or the like. The positive electrode current collector 12 may be constituted by a metal foil or a metal mesh. In particular, a metal foil is excellent in handling property and the like. The positive electrode current collector 12 may be made of a plurality of foils. Exemplary metals constituting the positive current collector 12 include Cu, Ni, Cr, Au, Pt, Ag, Al, Fe, Ti, Zn, Co, stainless steel, and the like. In particular, from the viewpoint of ensuring oxidation resistance and the like, the positive electrode current collector 12 may include Al. The positive electrode current collector 12 may have some coating layer on its surface for the purpose of adjusting the resistance or the like. Further, the positive electrode current collector 12 may be those in which the above metal is plated or deposited on a metal foil or a base material. When the positive electrode current collector 12 is made of a plurality of metal foils, some layer may be provided between the plurality of metal foils. The thickness of the positive electrode current collector 12 is not particularly limited. For example, it may be 0.1 µm or more or 1 µm or more, and may be 1 mm or less or 100 µm or less.

In addition to the above configuration, the positive electrode 10 may be provided with a general configuration as a positive electrode of an alkali metal ion battery. For example, a tab, a terminal, or the like. The positive electrode 10 can be manufactured by applying a known method. For example, the positive electrode active material layer 11 can be easily formed by molding a positive electrode mixture containing various components mentioned above in a dry or wet manner or the like. The positive electrode active material layer 11 may be molded together with the positive electrode current collector 12, or may be molded separately from the positive electrode current collector 12

### 2.2 Electrolyte Layer

The electrolyte layer 20 includes at least an electrolyte. The electrolyte layer 20 may include a solid electrolyte, and may further optionally include a binder or the like. When the electrolyte layer 20 includes the above alkali metal ion conductor of the present disclosure as an electrolyte, the electrolyte layer 20 may further include other electrolytes, binders, and various additives in addition to the alkali metal ion conductor. In this case, the content of the electrolyte, the binder, and the like in the electrolyte layer 20 is not particularly limited. Alternatively, the electrolyte layer 20 may include an electrolytic solution, and may further include a separator or the like for holding the electrolytic solution and preventing contact between the positive electrode active material layer 11 and the negative electrode active material layer 31. The thickness of the electrolyte layer 20 is not particularly limited, and may be, for example, 0.1 µm or more or 1 µm or more, and may be 2 mm or less or 1 mm or less.

The electrolyte contained in the electrolyte layer 20 may be appropriately selected from those exemplified as the above alkali metal ion conductor of the present disclosure and the electrolyte which may be contained in the above positive electrode active material layer. Further, the binder which may be included in the electrolyte layer 20 may be appropriately selected from those exemplified as the binder which may be included in the above positive electrode active material layer. Only one type of the electrolyte and the binder may be used alone, or two or more types thereof may be used in combination. When the alkali metal ion battery is an electrolytic solution battery, the separator for holding the electrolytic solution may be any separator commonly used in an alkali metal ion battery, and examples thereof include a separator made of a resin such as polyethylene (PE), polypropylene (PP), polyester, and polyamide. The separator may have a single layer structure or a multi-layer structure. Examples of the separator having a multi-layer structure include a separator having a two layer structure of PE/PP, or a separator having a three layer structure of PP/PE/PP or PE/PP/PE. The separator may be made of a nonwoven fabric such as a cellulose nonwoven fabric, a resin nonwoven fabric, or a glass fiber nonwoven fabric.

### 2.3 Negative Electrode

As shown in FIG. 1, the negative electrode 30 according to an embodiment may include a negative electrode active material layer 31 and a negative electrode current collector 32, and in this case, the negative electrode active material layer 31 may include the above alkali metal ion conductor.

### 2.3.1 Negative Electrode Active Material Layer

The negative electrode active material layer 31 includes a negative electrode active material, and may further optionally include an electrolyte, a conductive aid, a binder, and the like. Further, the negative electrode active material layer 31 may contain various other additives. When the negative electrode active material layer 31 contains the above alkali metal ion conductor of the present disclosure as an electrolyte, the negative electrode active material layer 31 includes a negative electrode active material in addition to the alkali metal ion conductor, and may further optionally include other electrolytes, conductive aids, binders, and various additives. The content of each of the negative electrode active material, the electrolyte, the conductive aid, the binder, and the like in the negative electrode active material layer 31 may be appropriately determined according to the battery performance. For example, the content of the negative electrode active material may be 40% by mass or more, 50% by mass or more, or 60% by mass or more, and may be less than 100% by mass or 90% by mass or less, taking the entire negative electrode active material layer 31 (entire solid content) as 100% by mass. The shape of the negative electrode active material layer 31 is not particularly limited, and may be, for example, a sheet shape having a substantially planar surface. The thickness of the negative active material layer 31 is not particularly limited. For example, it may be 0.1 µm or more, 1 µm or more, 10 µm or more, or 30 µm or more, and may be 2 mm or less, 1 mm or less, 500 µm or less, or 100 µm or less.

It is possible to employ, as the negative electrode active material, various materials in which a potential (charge/discharge potential) for occluding and releasing a predetermined ion is a base potential as compared with the above positive electrode active material. For example, a silicon-based active material such as Si, a Si alloy, or silicon oxide; a carbon-based active material such as graphite or hard carbon; various oxide-based active materials such as lithium titanate; metallic lithium, lithium alloy, or the like may be employed. Only one type of negative electrode active material may be used alone, or two or more types thereof may be used in combination. The shape of the negative electrode active material may be any general shape as the negative electrode active material of the alkali metal ion battery. For example, the negative electrode active material may be particulate. The negative electrode active material particles may be primary particles or secondary particles in which a plurality of primary particles are aggregated. The mean particle diameter (D50) of the negative active material particles may be 1 nm or more, 5 nm or more, 10 nm or more, and may be 500 µm or less, 100 µm or less, 50 µm or less, or 30 µm or less. Alternatively, the negative electrode active material may be a sheet (foil or film) such as a lithium foil. In other words, the negative electrode active material layer 31 may be made of a sheet of negative electrode active material.

Examples of the electrolyte that may be included in the negative electrode active material layer 31 include the above-described alkali metal ion conductor of the present disclosure, the above-described solid electrolyte, an electrolytic solution, or a combination thereof. Examples of the conductive aid which may be included in the negative electrode active material layer 31 include the above carbon material and the above metal material. The binder which may be included in the negative electrode active material layer 31 may be appropriately selected from those exemplified as the binder which may be included in the above positive electrode active material layer 11, for example. Only one type of the electrolyte and the binder may be used alone, or two or more types thereof may be used in combination.

### 2.3.2 Negative Electrode Current Collector

As shown in FIG. 1, the negative electrode 30 may include a negative electrode current collector 32 in contact with the above negative electrode active material layer 31. The negative electrode current collector 32 can employ any of common ones as a negative electrode current collector of a battery. Further, the negative electrode current collector 32 may be a foil, a plate, a mesh, a punching metal, a foam, or the like. The negative electrode current collector 32 may be a metal foil or a metal mesh, or may be a carbon sheet. In particular, a metal foil is excellent in handling property and the like. The negative electrode current collector 32 may be formed of a plurality of foils or sheets. Exemplary metals constituting the negative current collector 32 include Cu, Ni, Cr, Au, Pt, Ag, Al, Fe, Ti, Zn, Co, stainless steel and the like. In particular, from the viewpoint of ensuring reduction resistance and from the viewpoint of hardly alloying with an alkali metal, the negative electrode current collector 32 may be one containing at least one metal selected from Cu, Ni and stainless steel. The negative electrode current collector 32 may have some coating layer on its surface for the purpose of adjusting the resistance or the like. Further, the negative electrode current collector 32 may be those in which the above metal is plated or deposited on a metal foil or a base material. When the negative electrode current collector 32 is made of a plurality of metal foils, some layer may be provided between the plurality of metal foils. The thickness of the negative electrode current collector 32 is not particularly limited. For example, it may be 0.1 µm or more or 1 µm or more, and may be 1 mm or less or 100 µm or less.

In addition to the above configuration, the negative electrode 30 may be provided with a general configuration as a negative electrode of an alkali metal ion battery. For example, a tab, a terminal, or the like. The negative electrode 30 can be manufactured by applying a known method. For example, the negative electrode active material layer 31 can be easily formed by molding a negative electrode mixture containing the above various components in a dry or wet manner or the like. The negative electrode active material layer 31 may be molded together with the negative electrode current collector 32, or may be molded separately from the negative electrode current collector 32

### 2.4 Other Components

The alkali metal ion battery 100 may be those in which the above configurations is housed inside an exterior body. Any of the exterior bodies known as an exterior body of a battery can be employed as the exterior body. Further, a plurality of alkali metal ion batteries 100 are optionally electrically connected, also, optionally superimposed to obtain a battery assembly. In this case, the battery assembly may be housed inside a known battery case. The alkali metal ion battery 100 may be provided with an obvious configuration such as required terminals. The shape of the alkali metal ion battery 100 can include, for example, a coin, laminate, cylindrical, and rectangular cylindrical.

The alkali metal ion battery 100 can be manufactured by applying a known method. For example, it can be manufactured as follows. However, the method of manufacturing the alkali metal ion battery 100 is not limited to the following method, and each layer may be formed by, for example, dry molding or the like.
(1) A positive electrode active material or the like constituting the positive electrode active material layer is dispersed in a solvent to obtain a slurry for a positive electrode layer. The solvent used in this case is not particularly limited, and water or various organic solvents may be used. A positive electrode active material layer is formed on the surface of a positive electrode current collector by coating a slurry for a positive electrode layer on a surface of a positive electrode current collector using a doctor blade or the like and then drying the slurry, thereby forming a positive electrode.
(2) A negative electrode active material or the like constituting the negative electrode active material layer is dispersed in a solvent to obtain a slurry for a negative electrode layer. The solvent used in this case is not particularly limited, and water or various organic solvents may be used. A slurry for a negative electrode layer is coated on the surface of a negative electrode current collector using a doctor blade or the like, and then dried, whereby a negative electrode active material layer is formed on the surface of the negative electrode current collector, thereby forming a negative electrode.
(3) Each layer is laminated so as to sandwich an electrolyte layer (a solid electrolyte layer or a separator) between a negative electrode and a positive electrode, and a laminate having a negative electrode current collector, a negative electrode active material layer, an electrolyte layer, a positive electrode active material layer and a positive electrode current collector in this order is obtained. Other members such as terminals is attached to the laminate as necessary.
(4) The laminate is housed in a battery case, and in the case of an electrolytic solution battery, an electrolytic solution is filled in the battery case, and the laminate is immersed in the electrolytic solution, so that the laminate is sealed in the battery case, thereby forming an alkali metal ion battery. In the case of a battery containing an electrolytic solution, an electrolytic solution may be contained in the negative electrode active material layer, the separator, and the positive electrode active material layer at the above stage (3). As the electrolytic solution, a salt constituting the alkali metal ion conductor of the present disclosure may be used.

### 3. Method for Manufacturing Alkali Metal Ion Conductor

The alkali metal ion conductor of the present disclosure can be manufactured, for example, by mixing a plurality of types of salts. Specifically, the method for manufacturing an alkali metal ion conductor of the present disclosure may include mixing at least one tetraalkylammonium salt with at least one alkali metal salt and, in this case, the tetraalkylammonium salt has an alkyl chain length of 5 or more and has at least one first anion selected from the group consisting of a bromine ion, a chlorine ion, and a hydrogen sulfate ion. The alkali metal ion conductor of the present disclosure may be a mixture of a solid tetraalkylammonium salt and a solid lithium salt, which melts immediately after mixing.

### 3.1 Tetraalkylammonium Salt

The tetraalkylammonium salt has the above tetraalkylammonium ion as the first cation, and at least one of a bromine ion, a chlorine ion and a hydrogen sulfate ion as the first anion. Details of the first cation and the first anion are as mentioned above.

### 3.2 Alkali Metal Salt

The alkali metal salt has the above alkali metal ion as the second cation. The alkali metal salt may be, for example, a lithium salt. The anion constituting the alkali metal salt may be the above first anion, or may be a second anion different from the first anion. For example, the alkali metal salt may have at least one second anion selected from the group consisting of a sulfonyl amide anion and a complex ion containing H. Details of the second anion are as mentioned above.

### 3.3 Mixing Ratio

The mixing ratio of the tetraalkylammonium salt to the alkali metal salt is not particularly limited. For example, they may be mixed at a mixing ratio (second cation/first cation) so that the molar ratio of the second cation to the first cation is 0.05 or more and 19.0 or less, after mixing. The molar ratio may be 0.1 or more, 0.2 or more, 0.3 or more, 0.4 or more, 0.5 or more, 0.6 or more, 0.7 or more, 0.8 or more, 0.9 or more, or 1.0 or more, and may be 10.0 or less, 9.5 or less, 9.0 or less, 8.5 or less, 8.0 or less, 7.5 or less, 7.0 or less, 6.5 or less, 6.0 or less, 5.5 or less, or 5.0 or less.

### 3.4 Mixing Means

The salts constituting the alkali metal ion conductor of the present disclosure can be melted as a mixed salt by simply contacting the above tetraalkylammonium salt and alkali metal salt. The contact temperature may be 40°C or lower, 30°C or lower, or 20°C or lower. The mixing means is not particularly limited, and various mechanical mixing means can be used.

### EXAMPLES

As mentioned above, while an embodiment of the alkali metal ion conductor, the alkali metal ion battery and the method for manufacturing an alkali metal ion conductor of the present disclosure has been described, the alkali metal ion conductor, the alkali metal ion battery and the method for manufacturing an alkali metal ion conductor of the present disclosure can be variously modified other than the above-described embodiment without departing from the gist thereof. Hereinafter, the technique of the present disclosure will be described in further detail by way of Examples, but the technique of the present disclosure is not limited to the following Examples.

### 1. Fabrication of Mixed Salt

An organic salt shown in Table 1 below and a lithium salt as an alkali metal salt shown in Table 1 below were mixed in a mortar so that the molar ratio of the organic cation to the lithium ion (organic cation: lithium ion) was the molar ratio shown in Table 1 below, thus obtaining a mixed salt.

### 2. Conformation of Melting Temperature

The mixed salt thus obtained was charged in a glass screw bottle, and the bottle was placed on a hot plate in a state where the bottle is covered with a metal heat-retaining cover. The temperature of the hot plate was raised by 10°C at a time and, while maintaining at each temperature for 15 minutes, temperature rising was continued until the temperature reached a level at which the mixed salt was completely dissolved. The temperature at which the mixed salt was completely melted was specified as the melting temperature of the mixed salt. The results are shown in Table 1 below. When the mixture is melted at the time of mixing in the mortar, the melting temperature is lower than 20°C.

### 3. Measurement of Lithium Ionic Conductivity

The ionic conductivity at 25°C was measured for some of the mixed salts. Specifically, the mixed salt was poured into an electrolyte conductivity measurement cell made of PEEK, and the ionic conductivity was calculated from the resistance value determined from the room temperature impedance with SUS electrodes on both ends and the shape factor. The results are shown in Table 1 below.

**[Table 1]**

| | Organic salt | | | Li salt | | | Molar ratio | Melting temperature of mixed salt (°C) | Ionic conductivity (S/cm) |
|---|---|---|---|---|---|---|---|---|---|
| | Cation | Anion | Melting point (°C) | Cation | Anion | Melting point (°C) | | | |
| Example 1 | THA⁺ | Br⁻ | 99 | Li⁺ | TFSA⁻ | 240 | 50:50 | <20 | 0.192 |
| Example 2 | TOA⁺ | Br⁻ | 97 | Li⁺ | TFSA⁻ | 240 | 50:50 | <20 | 0.063 |
| Comparative Example 1 | TBA⁺ | TFSA⁻ | 95 | Li⁺ | TFSA⁻ | 240 | 50:50 | 70 | - |
| Comparative Example 2 | TEA⁺ | Br⁻ | 285 | Li⁺ | TFSA⁻ | 240 | 50:50 | 70 | <10⁻¹⁰ |
| Comparative Example 3 | TBA⁺ | I⁻ | 147 | Li⁺ | TFSA⁻ | 240 | 50:50 | 70 | <10⁻¹⁰ |
| Comparative Example 4 | TOA⁺ | I⁻ | 129 | Li⁺ | TFSA⁻ | 240 | 50:50 | 90 | <10⁻¹⁰ |
| Comparative Example 5 | THA⁺ | I⁻ | 104 | Li⁺ | TFSA⁻ | 240 | 50:50 | 60 | <10⁻¹⁰ |
| Comparative Example 6 | TBA⁺ | Br⁻ | 103 | Li⁺ | TFSA⁻ | 240 | 50:50 | 90 | <10⁻¹⁰ |
| Comparative Example 7 | EDMPhE⁺ | TFSA⁻ | 25 | Li⁺ | TFSA⁻ | 240 | 50:50 | 70 | 0.006 |
| Comparative Example 8 | PP₁₃⁺ | TFSA⁻ | 12 | Li⁺ | TFSA⁻ | 240 | 50:50 | 70 | 0.046 |
| Comparative Example 9 | AmTEA⁺ | TFSA⁻ | -2 | Li⁺ | TFSA⁻ | 240 | 50:50 | 50 | 0.032 |
| Comparative Example 10 | MTOA⁺ | TFSA⁻ | -75 | Li⁺ | TFSA⁻ | 240 | 50:50 | 50 | 0.028 |
| Example 3 | TAmA⁺ | Br⁻ | 101 | Li⁺ | TFSA⁻ | 240 | 50:50 | 30 | 0.245 |
| Example 4 | TDcA⁺ | Br⁻ | 88 | Li⁺ | TFSA⁻ | 240 | 50:50 | <20 | 0.035 |
| Example 5 | TDcA⁺ | Br⁻ | 88 | Li⁺ | TFSA⁻ | 240 | 20:80 | <20 | 0.013 |
| Example 6 | TAmA⁺ | Cl⁻ | 195 | Li⁺ | TFSA⁻ | 240 | 50:50 | <20 | 0.330 |
| Example 7 | THA⁺ | Cl⁻ | 113 | Li⁺ | TFSA⁻ | 240 | 50:50 | <20 | 0.232 |
| Example 8 | THA⁺ | HSO₄⁻ | 100 | Li⁺ | TFSA⁻ | 240 | 50:50 | <20 | 0.194 |
| Example 9 | THA⁺ | HSO₄⁻ | 100 | Li⁺ | TFSA⁻ | 240 | 20:80 | <20 | 0.105 |
| Example 10 | THA⁺ | HSO₄⁻ | 100 | Li⁺ | TFSA⁻ | 240 | 10:90 | <20 | 0.041 |
| Example 11 | THA⁺ | Br⁻ | 99 | LiGaCl₂I₂ | | 95 | 20:80 | <20 | 0.190 |
| Example 12 | THA⁺ | HSO₄⁻ | 100 | Li(CB₉H₁₀)_{0.5}(CB₁₁H₁₂)_{0. 5} | | None | 10:90 | <20 | - |
| Example 13 | THA⁺ | HSO₄⁻ | 100 | Li(CB9H10) | | None | 20:80 | <20 | - |
| Example 14 | THA⁺ | HSO₄⁻ | 100 | Li(CB9H10) | | None | 10:90 | <20 | - |
| Example 15 | THA⁺ | HSO₄⁻ | 100 | LiAlCl₂I₂ | | 70 | 10:90 | <20 | - |

Details of each cation and anion shown in Table 1 are as follows. The lithium salt of Example 11 contains a lithium ion and a gallium ion as cations, and a chlorine ion and an iodine ion as anions. The lithium salts of Examples 12 to 14 have a lithium ion as cations and a complex ion containing H such as (CB₉H10)⁻ and (CB₁₁H₁₂)⁻ as anions. The lithium salt of Example 15 contains a lithium ion and an aluminum ion as cations, and a chlorine ion and an iodine ions as anions.

### (Cation)

THA⁺ : Tetrahexylammonium ion, N(C₆H₁₃)₄⁺
TOA⁺ : Tetraoctylammonium ion, N(C₈H₁₇)₄⁺
TBA⁺ : Tetrabutylammonium ion, N(C₄H₉)₄⁺
TEA⁺ : Tetraethylammonium ion, N(C₂H₅)₄⁺
TAmA⁺ : Tetraamylammonium ion, N(C₅H₁₁)₄⁺
TDcA⁺ : Tetradecylammonium ion, N(C₁₀H₂₁)₄⁺
EDMPhE⁺ : Ethyldimethylphenylethyl ion, N(CH₃)₂(C₂H₅)(C₂H₅C₆H₅)⁺
PP₁₃⁺ : 1-Methyl-1-propylpiperidinium ion, (CH₃)(C₃H₇)N(C₅H₁₀)⁺
AmTEA⁺ : Amyltriethylammonium ion, N(C₂H₅)₃(C₅H₁₁)⁺
MTOA⁺ : Methyltrioctylammonium ion, N(CH₃)(C₈H₁₇)₃⁺

### (Anion)

TFSA⁻ : Trifluoromethanesulfonylamide ion, (CF₃SO₂)₂N⁻
Br⁻ : Bromine ion
I⁻ : Iodine ion
Cl⁻ : Chlorine ion
HSO₄⁻ : Hydrogen sulfate ion

In the above Examples, the case of employing a lithium salt as an alkali metal salt was illustrate as an example, but the type of the alkali metal salt is not limited thereto. It is considered that the effect of lowering the melting point by the tetraalkylammonium salt is exhibited regardless of the type of the alkali metal. In other words, the technology of the present disclosure is applicable not only to a lithium ion conductor containing a lithium salt, but also to various alkali metal ion conductors such as a sodium ion conductor containing a sodium salt and a potassium ion conductor containing a potassium salt.

### 4. Evaluation Results

From the above results, it can be said that an alkali metal ion conductor containing a salt (mixed salt) satisfying the following requirements has lithium ionic conductivity and has a low temperature (melting temperature) at which it behaves as a liquid.

(1) The salt contains a first cation, a second cation and a first anion.
(2) The first cation is a tetraalkylammonium ion having an alkyl chain length of 5 or more.
(3) The second cation is an alkali metal ion.
(4) The first anion is at least one anion selected from the group consisting of a bromine ion, a chlorine ion, and a hydrogen sulfate ion.

### REFERENCE SIGNS LIST

10 Positive electrode
11 Positive electrode active material layer
12 Positive electrode current collector
20 Electrolyte layer
30 Negative electrode
31 Negative electrode active material layer
32 Negative electrode current collector
100 Alkali metal ion battery

## Claims

1. An alkali metal ion conductor comprising a salt, wherein
the salt comprises a first cation, a second cation, and a first anion,
the first cation is a tetraalkylammonium ion having an alkyl chain length of 5 or more,
the second cation is an alkali metal ion, and
the first anion is at least one anion selected from the group consisting of a bromine ion, a chlorine ion, and a hydrogen sulfate ion.

2. The alkali metal ion conductor according to claim 1, wherein
the alkali metal ion is a lithium ion.

3. The alkali metal ion conductor according to claim 1 or 2, wherein
the salt comprises a second anion, and
the second anion is at least one anion selected from the group consisting of a sulfonylamide anion and a complex ion containing H.

4. An alkali metal ion battery comprising a positive electrode, an electrolyte layer, and a negative electrode, wherein
at least one of the positive electrode, the electrolyte layer and the negative electrode comprises the alkali metal ion conductor according to any one of claims 1 to 3.

5. A method for manufacturing an alkali metal ion conductor, which comprises:
mixing at least one tetraalkylammonium salt with at least one alkali metal salt, wherein
the tetraalkylammonium salt has an alkyl chain length of 5 or more, and comprises at least one first anion selected from the group consisting of a bromine ion, a chlorine ion, and a hydrogen sulfate ion.

6. The manufacturing method according to claim 5, wherein
the alkali metal salt is a lithium salt.

7. The manufacturing method according to claim 5 or 6, wherein
the alkali metal salt comprises at least one second anion selected from the group consisting of a sulfonylamide anion and a complex ion containing H.
